# EUROPEAN PATENT APPLICATION

(11) **EP 2 033 582 A2**
(43) Date of publication of application: **11.03.2009**
(21) Application number: 08172241.5
(22) Date of filing: 21.01.2005
(51) Int. Cl.: A61B 17/04, A61B 18/14, A61B 17/32

(54) **Tissue fastening and cutting tool, and methods**

(30) Priority: 23.01.2004 US 538616 P
(62) Divisional of application: 05711725.1
(71) Applicant: AMS Research Corporation, Minnetonka, MN 55343 (US)
(72) Inventor: Montpetit, Karen, Pilney, Minnesota, 55118 (US)
(74) Representative: Vossius & Partner

(57) **Abstract**

Disclosed are surgical instruments for manipulating soft tissue, by severing and making an attachment; embodiments of the surgical instruments can be capable of probing tissue, severing tissue, and making an attachment to the tissue such as attaching a suture.

## Description

### Field of the Invention

The invention relates to surgical instruments for manipulating tissue, and specifically relates to tools that are useful to sever soft tissue, connective tissue, visceral organs (e.g., uterus), etc., attach a fastener to the same tissue (before or after cutting the tissue), and optionally re-attach the severed tissue to a second tissue. The invention also relates to methods of using the tool to perform surgical procedures.

### Summary

Many surgical procedures require severing tissue, tissue manipulation, optionally attaching a fastener or marker to the tissue such as a suture, and desirably attaching the severed tissue to another tissue. In many instances, the manipulation is of tissue in a remote or difficult-to-access region of a patient's body. Procedures involving severing, manipulation, or attachment of a fastener or marker to a tissue are generally more difficult when operating in an inaccessible, highly sensitive, or remote region of the patient. Certain types of pelvic floor repairs, for example, may involve only transvaginal access to internal tissue through a relatively small incision. An example is tissue involved in pelvic floor repairs such as a sacrospinous ligament (used to attach to the vaginal vault for vault suspension) and cervical tissue in a transvaginal hysterectomy. An exemplary surgical procedure involving pelvic floor repair may include placement of a fastener such as a suture through the coccygeus muscle, uterosacral ligament, or sacrospinous ligament complex transvaginally (through a vaginal incision), e.g., during prolapse repair or a hysterectomy procedure for attachment of such tissue for vaginal vault suspension. Other exemplary surgical procedures can involve severing other connective tissue as well as tissue of visceral organs such as the uterus, at the cervical complex.

Transvaginal hysterectomies are being performed about one third as often as abdominal hysterectomies. Advantages of a transvaginal hysterectomy (relative to abdominal hysterectomies) are that the transvaginal procedure is less invasive (which correlates to fewer morbidities), requires shorter patient recovery, and can be combined with prolapse procedures. The transvaginal procedure, however, is more technically challenging due to limited exposure and access to the cervical complex and connective tissues (uterosacral, cardinal, and broad ligaments) and uterus.

During a hysterectomy, it can be desirable to attach one or more of the uterosacral, cardinal, and round ligaments to the vaginal cuff to support the vaginal vault following the procedure and reduce the possibility of subsequent prolapse. Attaching the uterosacral ligament, for example, to the vaginal cuff, requires the surgeon to sever the uterosacral ligament, which is contained in the uterosacral folds and which connects the uterus to the sacrum. With a transvaginal procedure, the uterosacral ligaments can be difficult to access. Furthermore, most gynecologist agree that grabbing the uterosacral ligaments at a high position facilitates the ability to provide adequate vault support once the attachment is made. Often, once these ligaments are severed, these ligaments will normally retract into the pelvic cavity and become difficult to retrieve, which is why many surgeons opt not to pursue transvaginal hysterectomies.

Medical practitioners are continually in need of new tool designs to more safely and efficiently perform surgical procedures. A tool specifically designed to improve safety and efficiency of a surgical procedure, e.g., a transvaginal hysterectomy or other procedure relating to the pelvic floor, can reduce the difficulty and technical challenges of the procedure and encourage surgeons to perform otherwise difficult procedures, as well as provide better patient outcomes and recovery.

### Summary

The invention is directed to surgical instruments and methods for use in soft tissue manipulation, severing, attachment, connection, "tagging," marking, or repair, including but not limited to procedures encountered in urological and gynecological applications. Instruments according to the invention may be useful in any surgical procedure where cutting tissue, removing tissue, or optionally making an attachment to a tissue, and optionally re-attaching the tissue to another tissue or a surgical implant, are desired. The instruments can be particularly suitable for use in soft tissue repair where the tissue is difficult to access, such as pelvic floor reconstruction procedures, especially those performed transvaginally.

Surgical instruments according to the invention are designed for and capable of the combined functions of grasping tissue, severing tissue for removal or re-attachment, and attaching a fastener to tissue. Optionally and preferably, the tool also allows re-attachment of the fastener or severed tissue to a second desired tissue or to another material such as a surgical implant. These combined functions allow a surgeon to grasp a desired portion of tissue, sever the tissue at a desired location, and attach a fastener to the tissue, all by use of a single tool and with only a relatively singular operation or series of steps that place the jaws of the tool at a single desired location or at multiple nearby locations, and actuate the cutting mechanism and the fastener-attaching mechanism in a desired order. Certain embodiments of the invention include a subsequent step of re-attaching the severed tissue to a second tissue or other material without removal of the tool from the surgical site.

Different types of fasteners that may be attached to a tissue include a suture, for example passing a suture-and-dart assembly or a suture-and-needle assembly; a mechanical coil or crimping attachment; and the like. Suture-passing tools are known to be capable of passing a dart-and-suture assembly or a needle-and-suture assembly from one region (e.g. a dart or needle transport jaw) to another region (e.g. a dart or needle capturing jaw) of a tool. Optionally and preferably, a fastener-attaching mechanism (e.g., a suture-passing mechanism) can include a bi-directional mechanism capable of retaining the fastener (e.g., suture, needle, or dart) at the capturing jaw after the first pass, and then re-passing the fastener (e.g., suture, needle, or dart) from the capturing jaw back to the original transport jaw, preferably for the purpose of reattaching the fastener to a desired second tissue location or other material, e.g., without removal of the tool from the surgical site.

The instrument, according to certain embodiments, may be shaped and sized for particular application, insertion, and use in a remote space of the body, such as transvaginal insertion to access tissue in the region of the pelvic floor. For such applications, the jaws of the tool are compact and are located at the end of an elongate extension that places the jaws a distance from the operating mechanisms of the body and handle of the device. The elongate extension allows the jaws to be inserted through a small incision, e.g., transvaginal incision, and to be capable of reaching tissue that is otherwise difficult to access.

In the particular example of a hysterectomy, the uterus is removed and a desired step is to attach one or more of a surrounding ligament or ligaments to the remaining cervical tissue or vaginal cuff to support the cuff and vaginal vault following the procedure. (Optionally, the cutting mechanism of the tool described herein, can be used to perform the step of cutting the uterus at the cervix.) Useful ligaments may be, e.g., the uterosacral ligaments, the cardinal ligaments, and the round ligaments. Most desirably, the surgeon can attempt to cut these ligaments from the cervical complex at a location that will provide a desired amount of tissue for reattachment and anchoring to the vaginal vault, e.g., cervical ring. Once the uterosacral, cardinal, or round ligament is cut, however, the ligament often retracts deep into the pelvic cavity where the ligament can be difficult to locate and retrieve if a suture, marker, or other tagging mechanism is not attached and available to identify and retrieve the ligament. As a result, a useful step of such as procedure can be to cut one or more of the uterosacral, cardinal, or round ligament and tag the ligament (i.e., place an attachment at the ligament) to facilitate locating the ligament for subsequent use during the procedure.

A tool according to the invention allows a surgeon to grasp a desired tissue, such as a ligament or ligaments, attach a fastener to mark or tag the tissue, and to allow retrieval of a ligament tissue if the ligament retracts following cutting of the tissue. Using the same tool, and while grasping the ligament optionally either at the same location or at a nearby location, a surgeon is able to cut the ligament using a mechanical or energy-delivered cutting mechanism (e.g., using a mechanical blade or scissor mechanism, heat, radio frequency energy, microwave energy, laser energy, etc.). The tissue is now severed at a desired position and also includes a "tag," or fastener that prevents the tissue (e.g., ligament) from retracting into the pelvic cavity, or at least allows the surgeon to retrieve the tissue (e.g., ligament) in the case of such retraction.

Next, the attached fastener (e.g., suture) can be used to reattach the tissue to another material or tissue such as a surgical implant or tissue at the vaginal cuff or cervical ring.

The tool can be used to sever any desired tissue, such as a ligament, or in a hysterectomy to sever the uterus from the cervical tissue complex using the same cutting mechanism for removal.

In addition, the tool can include other optional features that may be useful in combination with the cutting mechanism and fastener-attaching mechanism, such as a light at or near the jaws; a suture-managing element located, e.g., at one or both sides of the body of the tool; moveable or adjustable jaws that may be malleable, pivoting, or ratcheting); reloadable jaws (e.g., with a cartridge); removable (e.g., disposable) jaws; modular construction; an articulating or malleable distal portion such as at the jaws or at a location along an extension between the body and the jaws of a tool; a lockout feature for one or more of opening or closing the jaws, actuating the cutting mechanism, actuating of the fastener-attaching mechanism; etc. Also, the tool or different components of a modular tool may be disposable or reusable.

In one aspect, the invention relates to a surgical tool comprising: a body portion; an extension portion extending from the body portion; a jaw assembly comprising first and second jaws at a distal end of the extension portion, each jaw comprising a tissue contacting surface; a jaw actuator capable of moving at least one of the first and second jaws relative to the other of the first and second jaws, at least one of the jaws being operatively associated with the jaw actuator for manipulation between open and closed jaw positions; a cutting mechanism at the jaw assembly; and a cutting mechanism actuator capable of operating the cutting mechanism to cut tissue held between the jaws; wherein the jaw assembly comprises a fastener-attaching mechanism that is activatable to attach a fastener to tissue held between the jaws.

Preferably, the fastener is a suture and wherein the fastener-attaching mechanism comprises a suture-attaching mechanism located at the jaw assembly.

Preferably, the fastener-attaching mechanism is a suture-passer capable of passing a suture assembly from the first jaw to the second jaw, wherein the first jaw comprises a channel sized to deploy the suture assembly, and the second jaw comprises a surface sized and shaped to capture the suture-and-dart assembly.

More preferably, the suture-passer is further capable of passing a suture assembly back again to the first jaw after passing the suture assembly from the first jaw to the second jaw.

More preferably, the suture-passer is located on the jaw assembly distally from the cutting mechanism.

Preferably, the cutting mechanism comprises a mechanical blade assembly.

Preferably, the cutting mechanism comprises an emitting source of electromagnetic energy.

More preferably, the cutting mechanism comprises an energy source selected from the group consisting of radio frequency energy, laser energy, microwave energy, and combinations thereof.

More preferably, the tool is attached to a remote source of the electromagnetic energy.

The tool is preferably sized and shaped to be used transvaginally or laparoscopically.

In another aspect, the invention relates to a method of performing a hysterectomy. The method includes providing a surgical tool such as is described herein, inserting the jaw assembly of the tool to grasp a ligament tissue, actuating the cutting mechanism to sever the ligament, and actuating the fastener-attaching mechanism to attach a fastener to the ligament. According to certain methods of the invention, the ligament can be selected from a uterosacral ligament, a cardinal ligament, and a round ligament.

The method preferably comprises the step of, after attaching the fastener to the ligament, attaching the fastener to cervical tissue to connect the ligament to the cervical tissue.

The method is preferably performed transvaginaly or laparoscopically.

In another aspect, the invention relates to a method of performing a hysterectomy by providing a surgical tool as described herein; inserting the jaw assembly of the tool to grasp a ligament, actuating the cutting mechanism to sever the ligament, actuating the fastener-attaching mechanism to attach a fastener to the ligament, and attaching the fastener to cervical tissue.

The method is preferably performed transvaginally or laparoscopically.

Yet another aspect of the invention relates to a method of severing tissue. The method includes providing a surgical tool as described herein, introducing the jaw assembly to a surgical site to grasp a first tissue, actuating the cutting mechanism to sever the first tissue, and actuating the fastener-attaching mechanism to attach a fastener to the first tissue. Optionally, the method further includes attaching the fastener to a second tissue.

The method is preferably performed transvaginally or laparoscopically.

Yet another aspect of the invention relates to a kit that includes a tool as described herein, and fasteners.

### Brief Description of the Drawings

All drawings are schematic and are not to scale.
Figure 1 illustrates a tool according to the invention.
Figures 2a through 2f illustrate various embodiments of a jaw assembly of tools of the invention.
Figures 3a through 3f illustrate steps of an exemplary surgical procedure according to the invention.
Figure 4 illustrates another embodiment of a jaw assembly of a tool of the invention.
Figures 5a through 5c illustrate steps of another exemplary surgical procedure according to the invention.

### Detailed Description

The following describes the invention according to certain presently preferred embodiments that are meant to be illustrative only and not limiting. Other embodiments will be apparent to those of ordinary skill in the surgical instrument arts in view of this description.

According to the invention, a surgical tool includes combined mechanisms for attaching a fastener to a selected tissue and cutting the same portion of tissue near the fastener. The cutting mechanism and fastener-attaching mechanism are located at one or two jaws of the tool that can be placed at the tissue for cutting and attaching a fastener. The jaws are located at the end of an elongate extension, distal from a body or handle and actuating mechanisms. The distal portion of the tool includes the jaws, and the proximal portion includes the body and handles. In a typical embodiment the handles can be actuated to close the jaws onto tissue (other closing mechanisms are also possible, such as a sliding mechanism).

The tool can be useful for surgical procedures that involve cutting a desired tissue such as a visceral organ for removal, or a tissue or ligament that is to be attended to for later use -- meaning for example that the tissue or ligament may be subsequently attached to another tissue or to a surgical implant such as synthetic or biologic graft material, or the like. The inventive tool, having features for cutting a tissue and also for attaching a fastener to a tissue, can facilitate performing both the cutting and the fastener-attaching functions by providing a surgeon with a single tool that performs both functions.

The invention is broadly applicable to any surgical procedure that may involve cutting and fastener-attachment. Exemplary procedures include hysterectomies, which may be performed abdominally, transvaginally, or laparoscopically. The invention can be particularly useful to sever and attach a fastener to tissue located in a difficult-to-access location. As an example, the tool can be useful in transvaginal procedures such as a transvaginal or laparoscopic hysterectomy, to sever and attach a fastener to a ligament such as a uterosacral, cardinal, or a round ligament. The suture can assist the surgeon by marking the ligament at various times during the procedure such as prior to severing the ligament, and then again for re-attachment of the ligament elsewhere later in the procedure. By attaching a suture or other fastener or marker to the ligament, the severed ligament may retract but does not become lost, because the ligament can be easily located using the attached fastener.

The jaws of the tool include a fastener-attaching mechanism that is useful to attach a fastener to a tissue. The fastener can be any useful type of fastener, as will be understood, e.g., a mechanical fastener such as a suture, a suture-and-dart assembly, a suture-and-needle assembly, a suture cinch, a coil fastener, a crimp-type fastener, etc. The fastener-attaching mechanism of the tool can be any useful type of mechanism, as will be understood to be useful, e.g., mechanical fastener-attaching mechanisms such as those for attaching a suture, a suture-and-dart assembly, a suture-and-needle assembly, a suture cinch, a coil fastener, a crimp-type fastener, etc. The type of fastener-attaching mechanism can be of a known type or of a type developed in the future. Specific fastener-attaching mechanisms are known, such as the type described, e.g., in United States patent application Serial No. 10/155,710, entitled "SURGICAL SUTURE PASSERS AND METHODS," filed May 24, 2002, and which describes tools and methods of attaching a suture-and-dart assembly to a tissue with a single-pass mechanism. Other types of fastener-attaching mechanisms as are discussed herein will also be useful, such as suture-passing mechanisms that allow for bi-directional passage of a suture. An example of mechanisms useful to pass a suture and needle assembly bi-directionally, optionally with multiple pass capability, are mechanisms that are commercially available in suture devices sold under the trade name ARTHROSEW, by Tyco Healthcare and United States Surgical. Still other fastener-attaching mechanisms can be other mechanisms such as bonding tissue via using bio-glues or heat application.

The jaws of the tool also include a cutting mechanism. In general, the cutting mechanism can be any cutting mechanism located at one or both of the jaws, that can sever a desired tissue to which a fastener can also be attached. The cutting mechanism may be located at any useful and desired portion of either jaw or at both jaws, e.g., either distally or proximally from the fastener-attaching mechanism relative to the body (e.g., handles) of the tool. For a tool that is designed to sever one or more of the uterosacral ligament, cardinal ligament, round ligament, etc., or to sever the uterus from the cervix, during a hysterectomy, the cutting mechanism may be located proximal to the handle portion of the tool relative to the fastener-attaching mechanism. According to certain such configurations, during use, after the tissue is severed, and after the fastener is attached e.g., to one of the ligaments such as the uterosacral ligament, the fastener is connected to that portion of the severed ligament that remains attached to the sacrum and is not attached to the portion of the severed ligament that remains attached to the uterus.

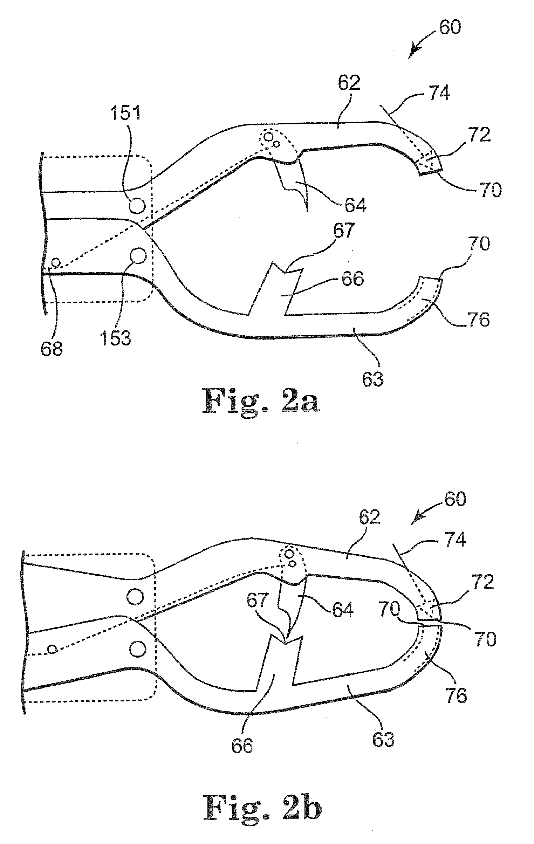

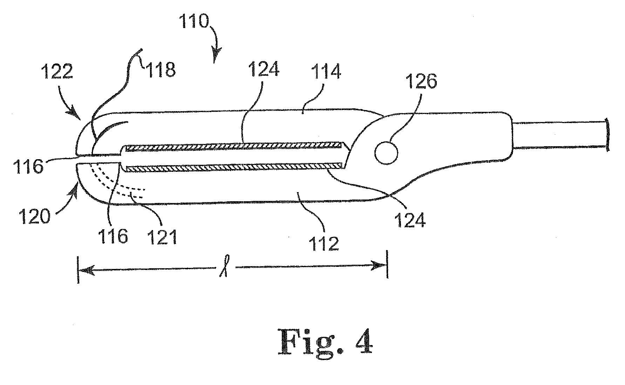

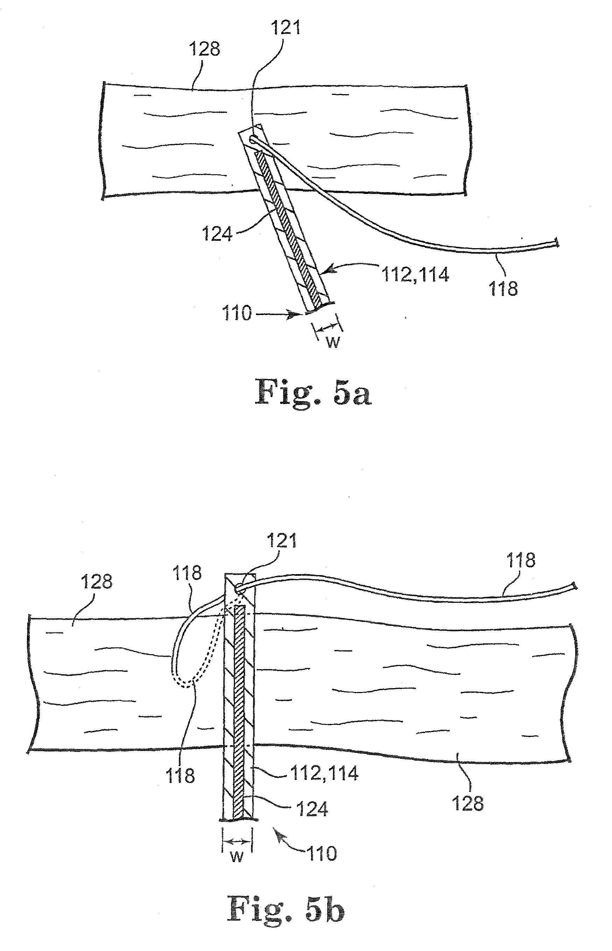

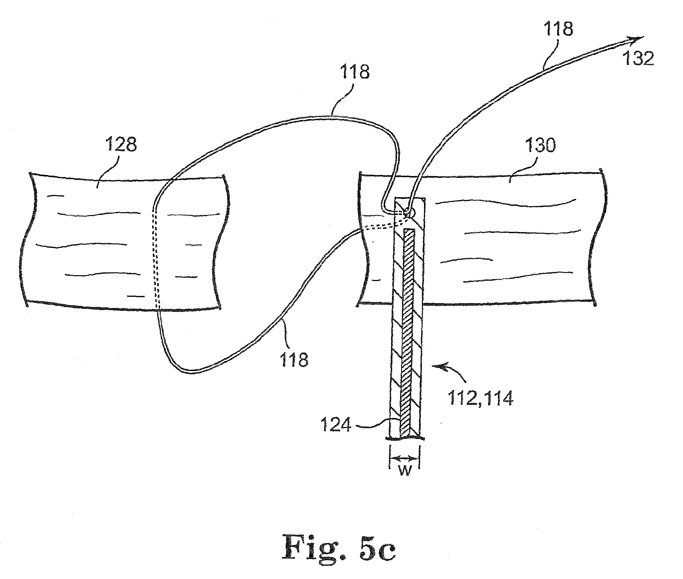

The jaws and cutting mechanism can be sized and shaped to cut a specific tissue. This can include a size to be useful for a particular surgical procedure, meaning, for example, overall jaw size that allows the jaws to access a particular tissue at a particular, possibly remote or difficult-to-access portion of the body. Further, the cutting mechanism can be shaped and sized (e.g., have an useful width or length) to allow the cutting mechanism to completely cut a specific tissue. The cutting mechanism may be situated and may be positioned to make a cut that extends along any direction relative to a set of jaws, such as along the width of a set of jaws (perpendicular to an axis of the tool extending from the body to the jaws, as in figures 2a through 2f), along a length of the jaws (parallel to an axis extending from the body to the jaws of the tool, e.g., as in figure 4, or at any other angle. (For purposes of the present discussion, the "length" of a jaw refers to the dimension that is parallel of the length-wise or longitudinal axis of the tool, e.g., the elongate extension; the "width" of the tool refers to the dimension that is perpendicular to the length and that, e.g., as illustrated in the attached figures, is also perpendicular to the direction of motion of the jaws when the jaws are actuated to open and close relative to one another. Figure 4 points out the length-wise dimension, "1," and figure 5, points out the width dimension, "w," of jaws of the illustrated tool.)

As an example of sizing of jaws and a cutting mechanism of a tool, embodiments of tools of the invention can be designed to be specifically useful to cut a uterosacral ligament, which may normally have a width in the range from 2 to 5 millimeters. Jaws and a cutting mechanism of the tool of the invention can be of a width or a length (depending on the orientation of the cutting mechanism along the width or length of the jaws) that is of a similar size range, to allow for complete severing of the ligament by a single actuation of the cutting mechanism. For tissues of larger or smaller sizes, the jaws may have a greater or smaller width or length (again, depending on the orientation of the cutting mechanism along the width or length of the jaws).

The jaws of a tool may each be separately moveable relative to the tool during opening and closing of the jaws, e.g., relative to the axis of an extension that connects the jaws to a body portion of the tool. Alternately, one of the jaws may be moveable and one may be stationary relative to the rest of the tool. Further, for jaws that are both moveable relative to the tool, both jaws may pivot relative to a single pivot point, or each jaw may pivot relative to a separate pivot point (each jaw has a different pivot point for a total of two pivot points). Jaws may be curved or straight. Straight jaws may have a parallel alignment (see figure 4), which may be preferred according to certain embodiments of the invention, such as embodiments that include a cutting mechanism that cuts using heat energy (e.g., RF, microwave, etc.), because parallel jaws may provide for uniform tissue compression and energy transfer during cutting. Figures 2a through 2f, for example, illustrate separate curved jaws that each move relative to the tool to open and close, and that each have a separate pivot point. Figure 4 illustrates an embodiment of a tool that has parallel jaws, wherein one jaw is stationary relative to the tool and a second jaw pivots relative to the tool and the first jaw.

The cutting mechanism may be any of various cutting mechanisms that are presently known or that may be developed in the future, and that can be placed at a jaw of a tool as described. For example, a useful cutting mechanism may be a mechanical mechanism such as a moveable (e.g., pivoting or retractable), sharp cutting surface (e.g., blade) located at a desired position to cut tissue held between two jaws of a tool. Other cutting mechanisms may be based on heat, electric current flow, ultrasound energy, or other forms of energy, such as a heating element, laser energy, microwave energy, radio frequency (RF) energy, or other electromagnetic energy capable of cutting tissue. Functional portions of these cutting mechanisms may be located at one or both jaws of the tool and may come into contact, alignment, or desired positioning for actuation to cut a tissue upon actuating the jaws to a closed position. Optionally, a cutting mechanism may be further moveable or pivotable relative to the jaws, if desired. Upon actuating (closing) the jaws, the cutting mechanism can be actuated to cut the tissue held between the cutting mechanism.

A cutting mechanism based on the use of heat, light, or other electromagnetic energy, can desirably be supplied by a source of cutting energy from a remote energy source, and actuated using a mechanical or other type of sensory switch to cause the energy to be emitted at the cutting surface or surfaces of the jaws. In such a system, a remote energy source may be located, e.g., in an operating room. A tool of the invention can be equipped with an adapter, e.g., at the body or handle of the tool, to receive the cutting energy from the remote source. The tool also has a transmitting mechanism to transfer the energy received from the adapter to the jaws and the cutting mechanism upon actuation of a cutting mechanism actuator.

The cutting mechanism can be actuated by a cutting mechanism actuator that can conveniently be located at the proximal portion of the tool, such as at the body or handle. The cutting mechanism actuator may be a type useful with a particular cutting mechanism. For a mechanical cutting mechanism such as moveable blades, a cutting surface or surfaces may be actuated to close and cut a tissue during actuation (closing) of the jaws. Alternately, the cutting mechanism may be actuated by use of a separate actuation mechanism not related to movement of the jaws, but that can be manipulated separately from the jaws and after the jaws have been closed.

For a mechanical cutting mechanism, the cutting mechanism actuator can be a mechanical actuator such as a rod or wire attached directly or indirectly to a moveable, extendable, or pivoting blade or cutting edge. When the jaws are in a closed position and the tool is ready for the cutting mechanism to be actuated, a rod or wire actuating mechanism can be moved by a cutting mechanism actuator (e.g., a sliding action, cam lever, knob, or trigger, etc.) located at a position of the tool for the operator to conveniently manipulate. The movement of the cutting mechanism actuator causes the blades to mechanically close and cut the tissue between the jaws and cutting mechanism. For a non-mechanical cutting mechanism, an actuator may be a switch, lever, knob, etc., that causes cutting energy such as heat, light, or other cutting energy, to be emitted at the cutting mechanism portion of the jaws of the tool to cause cutting of tissue located between the jaws.

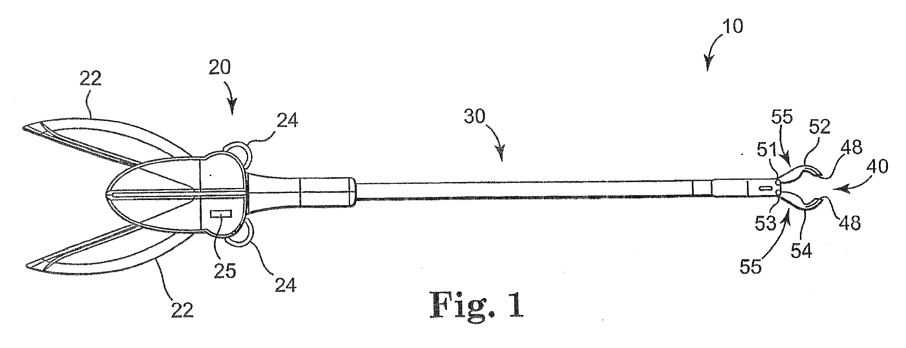

Referring to figure 1, there is shown an exemplary embodiment of a surgical device 10 according to the invention. The device 10 is suitable to perform the functions of grasping tissue, severing the tissue, and attaching a fastener to the tissue. Optionally and preferably, the device can further re-attach the severed tissue to a second tissue or to another material such as a surgical implant material, as desired. The different functions can be performed using separate or related motions of various controlling mechanisms and actuators located on the tool, e.g., on the body or handle portions of the tool.

Preferably, device 10 can also function to probe or test the integrity of tissue prior to actual severing of the tissue and prior to attachment of the fastener to the tissue. Accordingly, use of such a preferred embodiment of a tool allows for tissue to be tested for strength, character, robustness, or sufficient overall integrity, in a manner that does not cause the tissue to be damaged or traumatized, so the surgeon can determines that the tissue is appropriate for supporting a fastener and being-e.g., device 10 can be capable of providing tactile feedback to the surgeon concerning the integrity of probed tissue. Once suitable tissue is identified, the tissue can be severed and a fastener such as a suture can be attached to the tissue for subsequent use in connecting the tissue to other tissue or to another material such as a surgical implant.

The exemplary device 10 at figure 1 includes a body portion 20, a jaw portion 40 including first and second curved jaws 54 and 52, an elongate extension portion 30 projecting distally from the body portion 20, jaw manipulator (e.g. levers or handles) 22 for opening and closing jaws 52 and 54, fastener-attaching mechanism actuator (e.g., suture-attaching mechanism actuator) 24, and cutting mechanism actuator 25. Jaws 52 and 54 pivot between open and closed positions about pivots 51 and 53, coincidentally, upon actuation of jaw manipulator 22. Cutting mechanism 55 is shown generally as to location, with the location being at a portion of each of jaws 52 and 54 proximal to body portion 20 relative to the location of components of a fastener-attaching mechanism located to attach a fastener to tissue held between the tips 48 of jaws 52 and 54.

According to figure 1, fastener-attaching mechanism actuator 24 is shown to be mounted on the body portion 20 for movement relative to the body portion 20 between first and second positions. Functional portions of the fastener-attaching mechanism of the tool, which are not shown in detail, are located along the jaws 52 and 54, and may, for example, effect passage of a suture-and-dart assembly from the first jaw 52 to the second jaw 54, through tissue located between tips 48 of the jaws. Alternatively, a fastener-attaching mechanism actuator may be located elsewhere on the tool, such as at another location of the body, at a location of one or the other of jaw manipulators 22, or may be incorporated into the movement of the jaw manipulator 22.

Also according to figure 1, a cutting mechanism 55 is illustrated generically, meaning that details of a cutting mechanism are not shown, and the cutting mechanism may be any form of any cutting mechanism such as one that operates mechanically (e.g., mechanically operated blades) or one that functions based on heat energy, electromagnetic energy, microwave energy, ultrasound, laser or another form of energy useful for cutting tissue. Cutting mechanism actuator 25 is also illustrated generically, and may be any of various forms of actuating mechanism based on the type of cutting mechanism. Cutting mechanism actuator 25 may be, for example, a mechanical, electrical, or electronic button that can be depressed and released to alternate between actuated and non-actuated modes. If the cutting mechanism is based on the use of energy in the form of electromagnetic or heat energy, etc., an electrical or electronic switch may be preferred. Alternately, cutting mechanism actuator 25 may be, for example, a mechanical slide, knob, cam, or lever, that can be moved between actuated and non-actuated modes.

As illustrated, cutting mechanism actuator 25 can be located at the body portion 20 of the tool 10, within easy reach of either the operator's thumb or index finger, for easy manipulation during surgery. Alternatively, a cutting mechanism actuator may be located elsewhere on the tool, such as at another location of the body, at a location of one or the other of jaw manipulators 22, or may be incorporated into the movement of the jaw manipulator 22.

Still referring to figure 1, the fastener-attaching mechanism may be a suture-attaching mechanism, specifically able to pass a dart-and-suture assembly from one jaw, through tissue that is located between the jaws, to the other jaw. An example of such a suture-passing mechanism is described in Applicants' copending patent application serial number 10/155,710, entitled "SURGICAL SUTURE PASSERS AND METHODS", filed May 24, 2002. According to that type of fastener-attaching mechanism, actuator 24 can be manually actuated to actuate a dart cam located within the interior of jaw 52. When actuated, the dart cam moves to cause a suture-and-dart assembly to pass from jaw 52 to a receiving portion of jaw 54. As illustrated, the suture-passing mechanism actuator 24 includes a pair of firing members that are positioned so that they are within easy reach of either the surgeon's thumb or index finger. Also as shown in figure 1, the actuators 24 move in unison so that as the operator pulls back on one member, both members move back. Tool 10 may conveniently be fired with either the surgeon's index finger or thumb for advancement of the dart cam (described below).

A cutting mechanism of a tool of the invention may be any useful cutting mechanism, with various types of cutting mechanism contemplated as exemplary, e.g., at figures 2a through 2f, and figure 4. The jaws and cutting mechanisms of these illustrated embodiments of the invention can be of a size (e.g., length and width) and shape to cut tissue of a specific surgical procedure, preferably with a single actuation of the cutting mechanism.

For example, while the widths of the jaws and the cutting mechanism are not specified in figures 2a through 2f, the dimensions (e.g., width) can be suitable to allow the jaws to grasp a desired tissue (e.g., uterosacral ligament) and to also allow the cutting mechanism to span the tissue to cut the tissue with a single positioning step and a single step of actuating the cutting mechanism. More specifically, the cutting mechanisms of figures 2a through 2f are all perpendicular to the length of the jaws, i.e., the cutting surfaces align with the width of the jaws. This allows, the jaws to grasp tissue, attach a fastener to the tissue, and cut the tissue, without moving the jaws between the cutting and the fastener-attaching step. For the cutting step to be effective with only a single actuation of the cutting mechanism, the cutting mechanism can preferably span the entire width of the tissue while the tissue is being grasped by the jaws.

As comparison, figure 4 illustrates a set of jaws that include a cutting mechanism that is parallel to a length of the jaws, i.e., the cutting mechanism aligns with the length of the jaws. With these jaws, the jaws can grasp tissue at a first location of the jaws where the fastener-attaching mechanism is located (e.g., at the tips of the jaws). The fastener may then be attached to the tissue. Next, the jaws can be moved to place the tissue between the cutting mechanism along the length of the jaws, and the cutting mechanism can be actuated to cut the tissue. Again, for the cutting step to be effective with a single actuation of the cutting mechanism, the cutting mechanism (along the length of the jaws) can preferably span the entire width of the tissue while the tissue is located between the jaws at a location to be cut by actuation of the cutting mechanism. As illustrated in figure 4, the cutting mechanism is located along the length of jaws of the tool at a location proximal to the body portion of the tool relative to the fastener-attaching mechanism (which is located at the tips of the jaws). Alternately, the fastener-attaching mechanism could be located near the pivot point of the jaws, proximal to the body portion of the tool relative to the cutting mechanism. This configuration would still allow attachment of a fastener using the fastener-attaching mechanism near or toward the pivot portion of the jaws, followed by movement of the jaws to cut the tissue using the cutting mechanism portion of the jaws, distal from the fastener-attaching mechanism and along the length of the jaws.

Referring now to figures 2a and 2b, these figures illustrate an embodiment of moveable (closeable) curved jaws that include a suture-passing mechanism and a cutting mechanism. Jaws 62 and 63, having gripping surfaces 70, each pivot separately and coincidentally about pivots 151 and 153 by manipulation of an actuating mechanism (not shown). Once closed with tissue between the jaws, a suture-passing mechanism can pass a suture-and-dart assembly, 72 and 74, from the tip of jaw 62, through tissue (not shown), to the tip of the second jaw 63. The suture-passing mechanism (not shown in detail) includes a mechanism (e.g., a cam) that mechanically pushes suture-and-dart assembly (including suture 74 and dart 72) from the tip of jaw 62, through tissue between gripping surfaces 70, and to a receiving chamber 76 of jaw 63.

Jaws 62 and 63 of figure 2a additionally include a cutting mechanism made up of pivoting blade 64, fixed cutting surface 66, and wire 68. The cutting mechanism of this exemplary embodiment is aligned to cut tissue at an angle that is substantially perpendicular to the length-wise axis of the jaws. Wire 68 is attached to a cutting mechanism actuator at the proximal end of the tool (not shown). With tissue between gripping surfaces 70, blade 64 and cutting surface 67 line up to cut the tissue upon manipulation of wire 68 that causes blade 64 to pivot toward cutting surface 67. Thus, in use, as shown in figure 2b, jaws 62 and 63 can be closed; suture-and-dart assembly 72 and 74 can pass from jaw 62 to 63; and blade 64 can be actuated to sever tissue between the cutting surface of blade 64 and cutting surface 67.

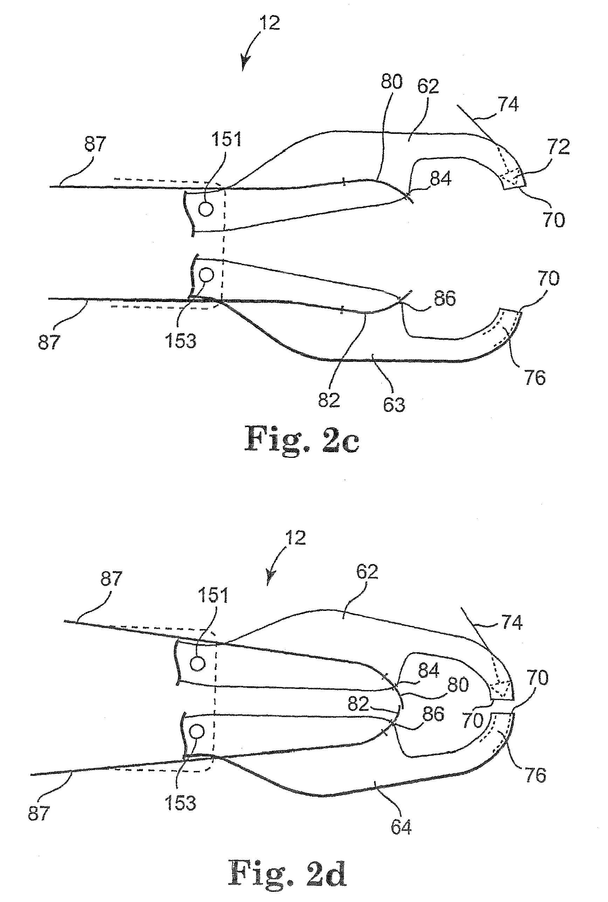

Another example of a mechanical cutting mechanism is shown at figures 2c and 2d, also with jaws 62 and 63 of an exemplary tool that include a suture-passing mechanism. Jaws 62 and 63, having gripping surfaces 70, pivot coincidentally about pivots 151 and 153 by manipulation of an actuating mechanism (not shown) at the proximal portion of the tool 12. Once closed with tissue between jaws 62 and 63, a suture-passing mechanism can pass a suture 74 from the tip of one jaw (62), through the tissue, to the tip of the second jaw (63). The suture-passing mechanism can include, e.g., a cam (not shown) within jaw 62 that pushes suture-and-dart assembly (including suture 74 and dart 72) from the tip of jaw 62, through tissue between gripping surfaces 70, and to a receiving chamber 76 of jaw 63.

Jaws 62 and 63 of figures 2c and 2d additionally include a cutting mechanism made up of blades 80 and 82, which can extend and retract through slots 84 and 86, by manipulation of actuating rods 87. (Again, the cutting mechanism of this exemplary embodiment is aligned to cut tissue at an angle that is substantially perpendicular to the length-wise axis of the jaws.) Rods 87 can be if a stiff metal or other rigid material capable of pushing blades 80 and 82 to extend through slots 84 and 86. At the proximal end of the tool (not shown), actuating rods 87 are attached to a cutting mechanism actuator (not shown). With tissue between gripping surfaces 70, blades 80 and 82 are actuated to extend to cut tissue in a direction that is substantially perpendicular to the length of the jaws (i.e., in a direction along the width of the jaws). Actuation of blades 80 and 82 is accomplished by manipulating actuating rods 87 to cause blades 80 and 82 to come together and sever the tissue (tissue not shown). Thus, in use, as shown in figure 2c and 2d, jaws 62 and 63 can be closed; suture-and-dart assembly 72 and 74 can pass from jaw 62 to 63 through a tissue between surfaces 70 of the jaws; and blades 80 and 82 can be actuated to sever tissue between the two blades.

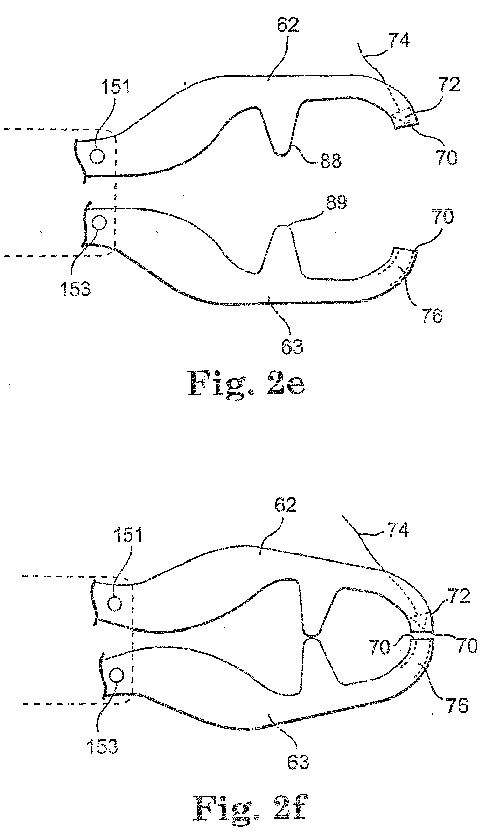

An example of a non-mechanical cutting mechanism (i.e., a cutting mechanism that functions based on electromagnetic or another form of cutting energy as opposed to a mechanical cutting action such as a blade) is shown at figures 2e and 2f, also with jaws 62 and 63 of a tool that include a suture-passing mechanism. Jaws 62 and 63, having gripping surfaces 70, pivot coincidentally about pivots 151 and 153 by manipulation of an actuating mechanism (not shown). Once closed with tissue between jaws 62 and 63, a suture-passing mechanism can pass a suture 74 from the tip of one jaw (62), through the tissue (not shown), to the tip of the second jaw (63).

Jaws 62 and 63 additionally include a cutting mechanism that include probes 88 and 89. Probes 88 and 89 will come together to contact tissue upon closure of jaws 62 and 63. Probes 88 and 89 then can emit cutting energy into or through the tissue to cause the tissue to be cut. Probes 88 and 89 may be separate (e.g., monopolar or bipolar) sources of cutting energy, which energy may be, e.g., radio frequency (RF), microwave energy, ultrasound, laser or other light energy, or any other suitable source of energy that can be used to cut tissue. The source of the energy can be remote, e.g., originating from a remote console that can be located in an operating room and attached to the tool for use during a surgical procedure. As illustrated, probes 88 and 89 are sized and shaped to contact the tissue when the jaws 62 and 63 are closed, and will be aligned to cut tissue at an angle that is substantially perpendicular to the axis of the jaws. Probes 88 and 89 could be activated electronically by an actuating mechanism (not shown) that can include electrical or electronic wiring, or optical fiber in the case of lasers, extending to the proximal end of the tool (not shown) and optionally to a remote energy source. With tissue between gripping surfaces 70 and probes 88 and 89, cutting energy radiates from probes 88 and 89 to cut the tissue. Thus, in use, as shown in figure 2e and 2f, jaws 62 and 63 can be closed; suture-and-dart assembly 72 and 74 can pass from jaw 62 to 63; and probes 88 and 89 are actuated to produce cutting energy to sever tissue between the two probes.

Figure 4 shows an example of a different variety of jaws of a tool of the invention. According to figure 4, jaws 112 and 114 of tool 110 are relatively straight and are parallel to each other and to the longitudinal axis of the tool when closed (as illustrated). This straight and parallel shape of the jaws can allow for good tissue contact along the length of jaws 112 and 114 when jaws 112 and 114 are closed over tissue. Tips 120 and 122 of jaws 112 and 114 can include a fastener-attaching mechanism such as any of those described herein. As illustrated, the fastener-attaching mechanism is shown to be a suture-passing mechanism, which can be a single-pass mechanism or, preferably, a bi-directional mechanism that is capable of passing a needle, suture, or dart from tip 122 of jaw 114 to tip 120 of jaw 112, and then back again, as desired. This can include passing a needle, as illustrated, using receiving chamber 121. The cutting mechanism of this exemplified tool includes two energy-emitting elements 124 that extend along the length of each of jaws 112 and 114. Elements 124 may emit cutting energy in the form of any one or more of microwave energy, RF energy, ultrasound energy, laser energy, etc. According to figure 4, jaw 112 is stationary and jaw 114 pivots about pivot point 126 to cause jaws 112 and 114 to open and close relative to one another.

As a further optional and sometimes preferred feature of a tool of the invention, the tool may be designed to attach a fastener to a first tissue, cut or sever the first tissue (these first two steps being performed in any useful order), and then re-attach the severed first tissue to a second tissue or to another material. As describe elsewhere herein, a fastener for attaching to a first tissue may be any of various forms of fasteners including a suture, a suture-and-dart assembly, a suture-and-needle assembly, a suture cinch, a coil fastener, a crimp-type fastener, etc. In a particular embodiment, a fastener may be a suture-and-needle assembly that can be passed from one jaw of the tool, through tissue (e.g., uterosacral ligament) and to the other jaw, where the needle can be held; the jaws can be opened and moved from the first (now severed) tissue, to a second tissue or another surgical material, and then the jaws can be closed to grasp the second tissue (e.g., cervical tissue) or material. The suture can be passed back from the receiving jaw, to the first jaw, through the second tissue or material, to connect the two tissues or the first tissue and the material. Thus, while not specifically illustrated, any of the embodiments of tools illustrated at figures 1 and 2a through 2f may embody a bi-directional suturing device with multiple pass capacity, in combination with any of the cutting mechanisms illustrated.

The tool of the invention can include still other optional features that will be understood to be useful, such as a light at or near the jaws to allow a surgeon to view the space of the cutting procedure. As another possibility, a tool may include a suture-managing element located, e.g., at one or both sides of the body of the tool. The suture-managing element can be a slit or a slot that contains a soft material such as a silicone, C-flex, foam, or another elastomeric material that provides a gripping force to hold a suture material at a desired position at the handle of the tool, during the surgical procedure. As other exemplary optional features the tool may include one or more of adjustable jaws (e.g., malleable, pivoting, or ratcheting jaws), reloadable jaws (e.g., with a cartridge) removable (e.g., disposable) jaws, modular construction, articulating distal portions (e.g., along an extension portion of the tool), a lockout feature, among other features. The tool may be disposable or reusable. Optionally, portions of a tool may be reusable (sterilizable) while other portions may be detachable and disposable -- a detachable, disposable jaw section will allow multiple uses of the non-disposable portion of the tool. A lockout feature may be provided to block either the fastener-attaching mechanism actuator or the cutting mechanism actuator unless the jaws are at a closed position. Optionally, the tool may be particularly sized and designed to be used laparoscopically.

Further, the jaw portion of a tool may be equipped with yet additional mechanisms that assist in severing tissue. As an example, a jaw portion may include a dispensing mechanism that is in communication with the proximal portion of the tool and that can dispense a liquid or other fluid at the cutting site. As a specific example, a tool may dispense an iodized solution such as saline, or the like, at the cutting site to irrigate the cutting site and facilitate uniform heat transfer along the jaws of a tool that uses an electromagnetic energy-producing cutting mechanism, e.g., in a bipolar mode.

The tool; components thereof; fasteners such as sutures, suture-and-dart assemblies, suture-and-needle assemblies, crimps, clips, or coils; and optionally a surgical implant such as a mesh material useful for treatment of vaginal vault prolapse; may be packaged together, separately, or in subassemblies depending on a variety of factors such as shelf life, sterilization requirements, and other preferences.

The surgical material or surgical implant may be any implantable surgical article, such as those used to treat conditions of vaginal vault prolapse. These article can include an implant made from mesh materials or a mesh material in combination with a synthetic or biological tissue material, connectors, etc. Various products are useful for treating conditions of vaginal vault prolapse, e.g., the APOGEE product from American Medical Systems. Very generally, these devices, e.g., "strips" of a single material or pieces of connected materials (e.g., mesh, tape, optionally including synthetic or biological tissue portions) are attached at one portion to the vaginal vault, then another portion of the strip is attached at a position of the anatomy to support the vaginal vault.

Any useful, convenient, or cost-effective sterilization procedure can be used to separately or collectively sterilize the contents of a kit, including methods that use steam, ethylene oxide, electron beam or other radiation, vapor (e.g., hydrogen peroxide or peracetic acid), plasma procedures, etc.

In another aspect, the invention relates to surgical methods, e.g., for soft tissue repair. A tool according to the invention can be designed for use in any of various surgical procedures that can benefit from attachment of a fastener such as a suture to tissue, in combination with cutting the tissue, optionally also in combination with grasping the tissue or probing the tissue for integrity. Examples of soft tissue repairs with which the invention may be particularly useful include pelvic floor reconstruction procedures, e.g., those that may be performed transvaginally, laparoscopically or abdominally. Examples of particular applications include but are not limited to uterosacral ligament fixation; closing cut vessels to provide any vessel or arterial ligation during vascular bypass or to provide hemostasis such as uterine artery ligation; vault prolapse repair; sacrospinous ligament fixation; paravaginal defect repairs; repairs of cystoceles, rectoceles, and enteroceles; prolapse repair; and deep pelvic suturing such as hypogastric arterial ligation, Oophorectomy, tubal ligation (fallopian tubes); and the like, e.g., performed transvaginally, laparoscopically, or otherwise.

In general, a method may include providing a device having first and second jaws movable between open and closed positions, a cutting mechanism also at the jaws, and a fastener-attaching mechanism. Certain preferred embodiments are presented for purposes of the present discussion, including a fastener-attaching mechanism in the form of a suture-passing mechanism for passing a suture between the jaws of the tool, through selected tissue, tissues, or a surgical material. Such a suture-passing mechanism may be for the single passage of a suture, or for multiple, bi-directional passage of a suture, which can allow for attachment of the suture to a tissue in combination with cutting the tissue, followed by re-attachment of the severed tissue to another portion of tissue or a surgical material.

Exemplary methods include loading a fastener (e.g., suture assembly) in the first jaw, probing tissue by moving the jaws between the open and closed positions at different locations of the tissue and attaching the fastener at the selected tissue. When the fastener is a suture, the suture can then be tightened through the tissue by withdrawing the device from the sutured tissue. Alternately, as discussed, a suture may be passed from the second jaw back through a different tissue, after which the suture can be tightened through both tissues by withdrawing the device. Before or after the fastener (e.g., suture) is attached to the first tissue, the tissue can be cut using the cutting mechanism, which may be mechanical or non-mechanical.

For certain preferred methods of the invention, the size and shape of the jaws and overall tool (e.g., elongate extension between jaws and a proximal body or handle) can be designed for insertion and operation in a remote region of the body, such as transvaginal insertion to access tissue in the region of the pelvic floor. Optionally, the size and shape may be designed for use laparoscopically. For such applications, the jaws of the tool are compact and may be sized to have a length and with that allow cutting of a particular body tissue. The compact and suitably-sized jaws are located at the end of an elongate extension that places the jaws a distance from the body, handles, and optional operating mechanisms at the proximal portion of the tool, which remains substantially external to the patient during surgery. The elongate extension allows the jaws to be inserted through a small incision, e.g., transvaginal incision, cannula, etc., and to be capable of reaching tissue that is otherwise difficult to access.

A hysterectomy procedure involves removal of the uterus by steps that include dissecting around the cervical ring. A surgeon may normally attempt to retain as much of the tissue of the cervical ring as possible. In the particular example of a transvaginal hysterectomy, the uterus is removed by cutting at the cervical ring and a desired step is to attach ligaments such as one or more of a uterosacral ligament, cardinal ligament, or round ligament, to the remaining cervical tissue or vaginal cuff, to provide support of for the vaginal vault. An important step of the procedure is cutting and tagging (i.e., placing an attachment at) one or more of these ligaments so a ligament can be located for later attachment to cervical tissue. Most desirably, the surgeon can attempt to cut this ligament at a location that will provide an amount of tissue that can be reattached to the vaginal vault, e.g., cervical ring. Once the ligament is cut, however, the ligament often retracts deep into the pelvic cavity where the ligament can be difficult to locate if a suture is not first attached to the ligament to identify and retract the ligament after retraction.

A method of the invention can involve using a tool as described herein to grasp a first tissue such as a ligament (e.g., uterosacral ligament) and attach a fastener to the tissue, e.g., a suture, clip, or coil, etc. For a ligament, the fastener functions to mark or tag the ligament or ligaments. Using the same tool, optionally while grasping the tissue at the same location, the surgeon can also cut the tissue using a mechanical or energy-derived cutting mechanism (e.g., using heat, RF energy, microwave energy, laser energy, etc.), also located at the jaws of the tool. The cutting step may be before or after the fastener-attaching step, depending on different features of the tool and preferred order during a surgical procedure. When applied to a ligament, the ligament now includes a "tag," or fastener that prevents the ligament from retracting into the pelvic cavity, or at least allows the surgeon to retrieve the ligament upon such retraction.

Next, the attached fastener (e.g., suture) can be used to reattach the tissue to a second tissue or to a surgical implant or other surgical material, as desired. Re-attachment of the severed and tagged tissue may be accomplished by any useful method. As an example, the tool may be retracted from the surgical site, and the suture may be re-installed into a second fastener-attaching device (e.g., a suture-passing device) and the fastener may be attached to a second tissue. Alternately, the tool may include a mechanism to retain a suture at the jaw to which the suture was passed during the first fastener-attaching step, so the suture can be directly reattached to the second tissue by re-passing the suture back to the first jaw.

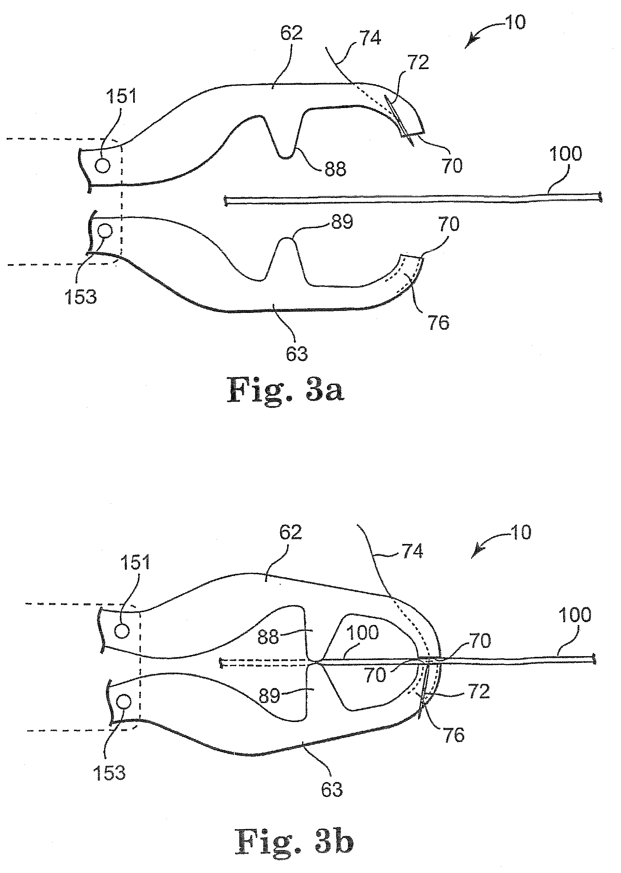

An illustrative, non-limiting example of a method of the invention is shown at figures 3a through 3e. The example is described in terms of a hysterectomy procedure that involves cutting the uterosacral ligament and re-attaching the uterosacral ligament to cervical tissue to support the vaginal cuff. It is to be understood, however, that similar steps can be performed to sever and attach a fastener to any desired tissue, for other types of surgical procedures, and to optionally re-attach the tissue to a second tissue or other surgical material.

Referring to figure 3a, a tool such as tool 10 is illustrated to have jaws that include a non-mechanical cutting mechanism and a bi-directional suture-passing mechanism. Jaws 62 and 63, having gripping surfaces 70, pivot about pivots 151 and 153 by manipulation of an actuating mechanism (not shown). Once closed with tissue between jaws 62 and 63, a suture-passing mechanism can pass a needle 72 and suture 74 from the tip of jaw 62, through the tissue 100, to the tip of jaw 63, at receiving cavity 76 (not to scale).

Jaws 62 and 63 include a cutting mechanism that includes probes 88 and 89, which come together to substantially contact tissue 100, including spanning of the width (not shown) of tissue 100, upon closure of the jaws. See figure 3b. Probes 88 and 89 can then be actuated to emit cutting energy into tissue 100 to cut the tissue 100.

Referring to figure 3a, the jaw portion of tool 10 is inserted to a surgical site to contact and grasp uterosacral ligament 100. As shown in figure 3b, jaws 62 and 63 are closed to cause each of jaw surfaces 70 to contact opposing surfaces of tissue 100, while also placing probes 88 and 89 into contact with opposing surfaces of tissue 100. A first mechanism of a bi-directional suture-passing mechanism (not shown in detail) is actuated to cause needle 72 and suture 74 to pass from jaw 62, through tissue 100, and to be received by receiving chamber 76 of jaw 72. Suture 74 passes through tissue 100. Before or after passing needle 72 and suture 74 pass through tissue 100, a cutting mechanism comprising probes 88 and 89 is actuated to sever tissue 100 at the location between probes 88 and 89. Tissue 100 is severed, needle 72 and suture 74 have been passed through severed tissue 100, and needle 72 has been received by and is held by a receiving mechanism of jaw 63.

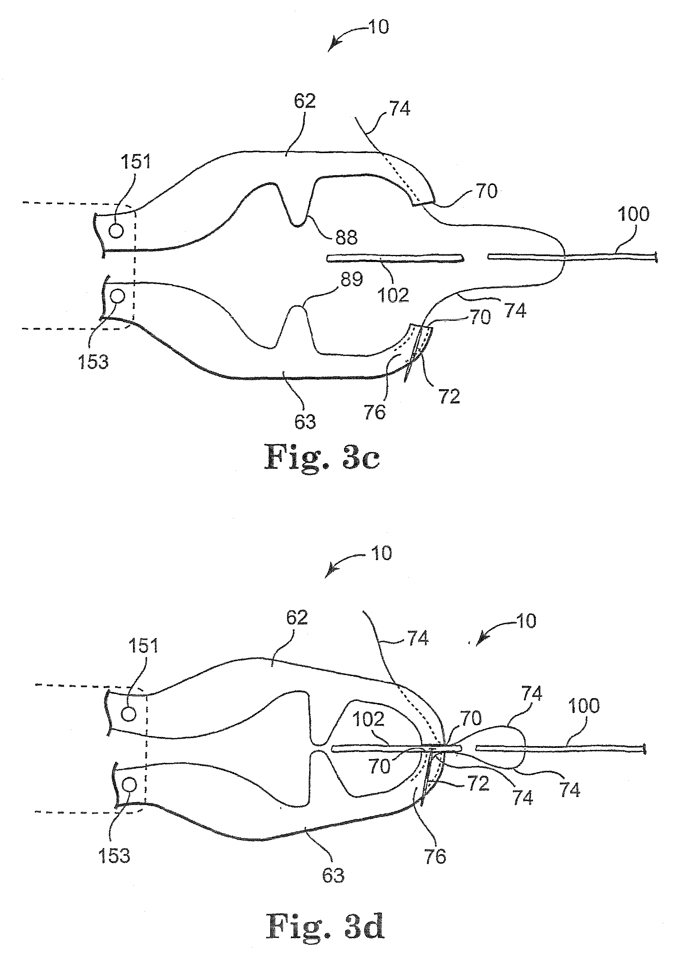

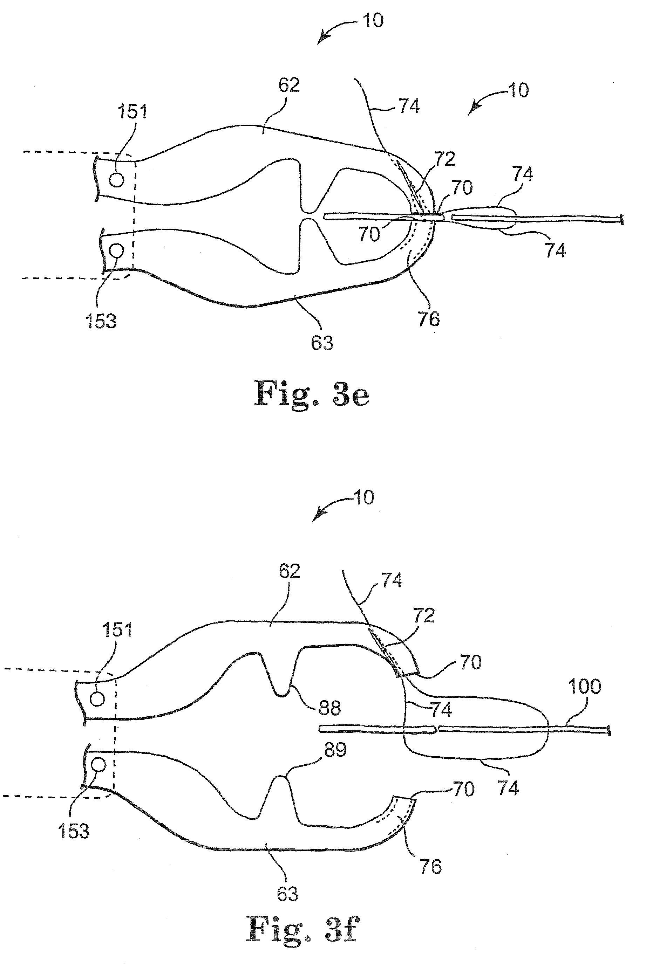

Referring to figure 3c, the illustrated embodiment of the invention allows for relatively direct re-attachment of suture 74 to a second tissue, illustrated as cervical tissue 102. According to the method of this embodiment, tool 10 is moved to place jaws 62 and 63 at a location to grasp cervical tissue 102. Suture 74 extends through tissue 100 and to needle 72. As shown in figure 3d, surfaces 70 of jaws 62 and 62 grasp cervical tissue 102, and needle 72 is ready to pass through cervical tissue 102 by use of a second component of the bi-directional suture-passing mechanism. Upon such actuation of the second component of the bi-directional suture-passing mechanism (not shown), needle 72 and suture 74 are passed from the tip of jaw 63, through cervical tissue 102, and back to jaw 62 where needle 72 is again received and held. See figure 3e. Next, as shown in figure 3f, jaws 62 and 63 are opened, tool 10 can be withdrawn, and suture 74 has can be tightened to secure tissue 100 to tissue 102.

In another variation of a method of the invention, a tool such as tool 110 of figure 4 can be used to cut tissue and attach a fastener to the tissue. Referring to figure 5a, jaws 112 and 114 of tool 110 are viewed in cross section (width of the jaws is indicated as "w"), as is the location of cutting elements 124 relative to each of jaws 112 and 114. As shown at figure 5a, this method includes a step of grasping tissue 128 using the tips of jaws 112 and 114, to allow a fastener (suture 118) to be attached to the tissue by passage of a needle (not shown) from upper jaw 114 to lower jaw 112. Jaws 112 and 114 can then be opened and moved to a different portion of the same tissue, causing suture 118 to extend while still being located through tissue 128 (and while the needle is held by jaw 112, to which the needle has been passed). As shown at figure 5b, jaws 112 and 124 can be closed to place cutting elements 124 to contact tissue 128, at which time cutting elements 124 can be actuated to cut tissue 128 where tissue 128 is contacted by elements 124. In figure 5a, suture 118 extends from a position external to the surgical site, 132, is threaded through the tip of jaw 114, runs through tissue 128, and then connects to the needle held at the tip of jaw 112, beneath the tissue.

Subsequent to this sequence for cutting tissue 128, tissue 128 may be reattached to a different tissue or a surgical material by any method. According to a particular method, wherein tool 110 includes a bi-directional suture-passing mechanism, jaws 112 and 114 can again be moved and closed to grasp a second tissue, 130. As shown in figure 5c, suture 118 extends from end 132 (outside of the surgical area) into and through upper jaw 114 (without yet passing through tissue 130), through tissue 128, and back to lower jaw 112 where the suture is held in the jaw with a needle. The needle or dart, and suture 118, are then passed through tissue 130 and received by upper jaw 114. Jaws 112 114 can be opened and the both ends of the suture can be brought out of the surgical space. The suture has been passed through both of tissues 128 and 130, and slack can be tightened.

## Claims

1. A surgical tool comprising:
a body portion;
an extension portion extending from the body portion;
a jaw assembly comprising first and second jaws at a distal end of the extension portion, each jaw comprising a tissue contacting surface;
a jaw actuator capable of moving at least one of the first and second jaws relative to the other of the first and second jaws, at least one of the jaws being operatively associated with the jaw actuator for manipulation between open and closed jaw positions;
a non-mechanical cutting mechanism located at the jaw assembly; and
a cutting mechanism actuator capable of operating the cutting mechanism to cut tissue held between the jaws;
wherein the jaw assembly comprises a fastener-attaching mechanism that is activatable to attach a fastener to tissue held between the jaws.

2. A surgical tool comprising:
a body portion;
an extension portion extending from the body portion;
a jaw assembly comprising first and second jaws at a distal end of the extension portion, each jaw comprising a tissue contacting surface;
a jaw actuator capable of moving at least one of the first and second jaws relative to the other of the first and second jaws, at least one of the jaws being operatively associated with the jaw actuator for manipulation between open and closed jaw positions;
a cutting mechanism located at the jaw assembly; and
a cutting mechanism actuator capable of operating the cutting mechanism to cut tissue held between the jaws;
wherein the jaw assembly comprises a bi-directional suture-passing mechanism capable of passing a suture assembly from the first jaw to the second jaw, and then passing the suture assembly back from the second jaw to the first jaw.

3. A surgical tool comprising:
a body portion;
an extension portion extending from the body portion;
a jaw assembly comprising first and second jaws at a distal end of the extension portion, each jaw comprising a tissue contacting surface;
a jaw actuator capable of moving at least one of the first and second jaws relative to the other of the first and second jaws, at least one of the jaws being operatively associated with the jaw actuator for manipulation between open and closed jaw positions;
a cutting mechanism located at the jaw assembly, the cutting mechanism comprising a blade pivotably connected to one of the first and second jaws; and
a cutting mechanism actuator capable of pivoting the blade of the cutting mechanism relative to the one of the first and second jaws to cut tissue held between the jaws;
wherein the jaw assembly comprises a fastener-attaching mechanism activatable to attach a fastener to tissue positioned between the jaws.

4. A tool according to claim 1 wherein the fastener is a suture and wherein the fastener-attaching mechanism comprises a suture-passing mechanism located at the jaw assembly.

5. The tool of claim 1 wherein the fastener-attaching mechanism is a suture-passing mechanism capable of passing a suture assembly from the first jaw to the second jaw, wherein
the first jaw comprises a channel sized to deploy the suture assembly, and
the second jaw comprises a surface sized and shaped to capture the suture-and-dart assembly.

6. The tool of claim 4 wherein the suture-passing mechanism is further capable of passing a suture assembly back again to the first jaw after passing the suture assembly from the first jaw to the second jaw.

7. A tool according to any of claims 2 through 6 wherein the suture-passing mechanism is located on the jaw assembly distally from the cutting mechanism.

8. The tool of claim 2 wherein the cutting mechanism comprises a mechanical blade assembly.

9. The tool of claim 1 wherein the non-mechanical cutting mechanism comprises an emitting source of electromagnetic energy.

10. A tool according to claim 2 comprising a non-mechanical cutting mechanism comprises an emitting source of electromagnetic energy.

11. A tool according to claim 9 or 10 wherein the non-mechanical cutting mechanism comprises an energy source selected from the group consisting of radio frequency energy, laser energy, microwave energy, and combinations thereof.

12. A tool according to claim 9 or 10 attached to a remote source of the electromagnetic energy.

13. A tool according to any of claims 1 through 12 sized and shaped to be used transvaginally or laparoscopically.

14. A tool according to any of claims 1 through 13 wherein the cutting mechanism comprises a cutting surface aligned to cut tissue at an angle substantially perpendicular to a length-wise axis of the jaw assembly.

15. A tool according to claim 2 or 8 wherein
the cutting mechanism comprises a blade pivotably connected to one of the first and second jaws; and
the cutting mechanism actuator is capable of pivoting the blade of the cutting mechanism relative to the one of the first and second jaws to cut tissue held between the jaws.
